# EUROPEAN PATENT SPECIFICATION

(11) **EP 2 722 069 B1**
(45) Date of publication and mention of the grant of the patent: **03.01.2018**
(21) Application number: 12801433.9
(22) Date of filing: 11.04.2012
(51) Int. Cl.: A61M 25/00, B05D 3/00, B05D 5/00, A61L 29/00, A61L 29/14, A61L 29/08, A61M 25/06

(54) **SHEATH FOR INTRODUCER AND INTRODUCER ASSEMBLY**
HÜLLE FÜR EINEN INSERTER UND INSERTERANORDNUNG
GAINE POUR INTRODUCTEUR ET ENSEMBLE INTRODUCTEUR

(30) Priority: 15.06.2011 JP 2011133601
(43) Date of publication of application: 23.04.2014
(73) Proprietor: Terumo Kabushiki Kaisha, Tokyo 151-0072 (JP)
(72) Inventor: OKAMURA, Ryo, Fujinomiya-shi Shizuoka 418-0015 (JP)
(74) Representative: Dossmann, Gérard
(86) International application number: PCT/JP2012/059908
(87) International publication number: WO 2012/172861

(56) References cited:
- WO-A1-2007/122908
- WO-A1-2007/143280
- WO-A2-97/44082
- WO-A2-98/55172
- WO-A2-99/44665
- WO-A2-2007/103772
- JP-A- 2003 510 134

## Description

### Technical Field

The present invention relate to a sheath for introducer, and an introducer assembly.

### Background

In recent years, in a medical treatment, there have been carried out various forms of medical treatments and checkups by using an elongated hollow & tubular medical instrument referred to as a catheter. As such a medical treatment method, there exists a method of administering medicine directly into a target lesion by utilizing the elongated property of the catheter, a method of pushing & widening and opening a stenosis region inside a body-cavity by using a catheter equipped with a balloon, which spreads by pressurization, at the distal end thereof, a method of shaving off and opening a target lesion by using a catheter equipped with a cutter at the distal portion thereof, conversely, a method of applying a filler and closing an aneurysm, a bleeding place or a feeding vessel by using a catheter, or the like. Also, there exists a medical treatment method in which in order to maintain a stenosis region inside a body-cavity in an opened state, a tube-shaped stent constituted to be in the shape of net for the side surface thereof is embedded and indwelled inside a body-cavity by using a catheter, or the like. Further, there exists a method of sucking a liquid which has become excessive for the internal body, or the like.

In case of carrying out a medical treatment & checkup or the like by using a catheter, generally, a sheath for introducer is introduced into a sticking region formed in an arm or a leg by using a catheter introducer and a catheter or the like is percutaneously inserted into a diseased region such as a blood vessel or the like through a lumen of the sheath for introducer.

The sheath for introducer is formed from a sheath tube which is a tubular member provided with a hollow portion through which an elongated body such as a catheter is freely insertable (see Patent Document 1 and 2). In the Patent Document 1, there is described a configuration of providing a hydrophilic coating on the inner surface of a sheath for introducer in order to reduce sliding resistance when inserting an elongated body such as a catheter through the sheath for introducer. Also, in the Patent Document 2 and 3, there is described a configuration of providing a hydrophilic coating on the outer surface of a sheath for introducer in order to secure the lubricity of the outer surface of the sheath for introducer. In this Patent Document 2, there is described a sheath for introducer in which the inner diameter at the distal portion thereof is formed to become gradually smaller toward the distal side (see FIG. 4 of Patent Document 2).

### Prior Art Document

### Patent Document

Patent Document 1:
   Japanese Unexamined PCT Patent Publication No. 2003-510134
Patent Document 2:
   Japanese Unexamined Patent Publication No. 2002-291902
Patent Document 3:
   WO 97/44082

### Summary of Invention

### Technical Problems

When the inner diameter of the distal portion is formed gradually in smaller size toward the distal side as described in the Patent Document 2, sliding resistance between the inner surface of the distal portion and elongated body such as a catheter becomes large and there occurs a phenomenon that slidability of the catheter or the like lowers. In order to improve such a situation, it is conceivable, as described in the Patent Document 1, that there is provided a hydrophilic coating on the inner surface of the sheath for introducer. However, the heat at the time of carrying out a shape-application process of the distal portion by using a die assembly acts excessively, and there occurs a phenomenon that the hydrophilic coating will peel, will decompose, will degrade or the like. Consequently, the sliding resistance between the inner surface of the distal portion and the elongated body such as a catheter does not lower substantially and there is such a problem that it is not possible to achieve improvement in the slidability of the catheter or the like.

Also, when pulling out the sheath for introducer after being inserted inside the blood vessel in a case in which there is provided hydrophilic coating on the outer surface of the sheath for introducer as described in the Patent Document 2, the hydrophilic coating already became wet caused by the blood, lubricity thereof occurs and it becomes a situation in which the friction resistance is small. Consequently, it is possible to pull out the sheath for introducer smoothly. However, at the beginning of the use of the sheath for introducer, the hydrophilic coating is under a dry condition, so that the sliding property is bad and the friction resistance thereof is large. Consequently, when inserting it from the skin toward the inside of the blood vessel, there is such a problem that the insertion resistance of the sheath for introducer is large.

Consequently, the present invention is addressed to provide a sheath for introducer in which improvement in the slidability of an elongated body such as a catheter or the like is achieved even in case of forming the inner diameter of the distal portion so as to become gradually smaller toward the distal side.

Also, the present invention is also addressed to provide a sheath for introducer in which reduction in insertion resistance is achieved even under a dry condition at the beginning of the use thereof.

### Technical Solution

The present invention achieved the abovementioned object by providing a sheath for introducer as defined by the claims.

### Advantageous Effect

According to the present invention, the inner diameter of the distal portion is formed to become gradually smaller toward the distal side and on the inner surface of the distal portion, there is provided with a hydrophobic coating having a friction coefficient lower than the friction coefficient of the tubular member. Consequently, even in case of forming the inner diameter of the distal portion gradually smaller toward the distal side, it is possible to provide a sheath for introducer in which improvement in the slidability of an elongated body such as a catheter or the like is achieved.

The outer surface of the distal portion is further provided with a hydrophobic coating. In this case, it is possible to achieve reduction in insertion resistance even under a dry condition at the beginning of the use thereof.

According to the present invention, there is provided with a hydrophilic lubricating coating on the outer surface of the main body portion and there is provided, on the outer surface of the distal portion, with a hydrophobic coating having a friction coefficient lower than the friction coefficient of the hydrophilic lubricating coating during the dry period thereof. Consequently, even under a dry condition at the beginning of the use thereof, it is possible to provide a sheath for introducer in which reduction in insertion resistance is achieved.

It is preferable for the hydrophobic coating to cover the upper portion of the hydrophilic lubricating coating at the boundary portion between the distal portion and the main body portion. In this case, when inserting the sheath for introducer from the skin into the inside of the blood vessel, it never happens that the sheath gets stuck on the end surface of the hydrophilic lubricating coating at the boundary portion. Consequently, it is possible to insert the sheath for introducer smoothly and it is possible to suppress peeling of the hydrophilic lubricating coating.

When absorbing the blood and swelling, it is preferable for the hydrophilic lubricating coating to get a diameter approximately equal to or greater than the outer diameter of the hydrophobic coating. In this case, when pulling out the sheath for introducer from the blood vessel, there does not occur a step at the overlapping portion in which the hydrophobic coating overlaps the hydrophilic lubricating coating. Consequently, the resistance of the surface of the sheath for introducer with respect to the blood vessel and the skin does not become surplus and it is possible to pull out the sheath for introducer smoothly owing to both the coatings.

According to the present invention, there is employed a constitution in which at least a portion of the inner surface of the distal portion of the sheath for introducer and a portion of the outer surface of the dilator are attached to each other through hydrophobic coating. In this introducer assembly, by attaching the distal portion and the dilator to each other through hydrophobic coating, it is possible to suppress a turnup of the distal portion when inserting the sheath for introducer and to insert the sheath for introducer smoothly.

It is preferable for the hydrophobic coating to be formed from a reactive-curing coating material and to be formed by reactively curing the coating material in a state in which the dilator is passed through the sheath for introducer. In this case, it is possible to carry out the formation of the hydrophobic coating and the attachment between the inner surface of the distal portion and the outer surface of the dilator through this hydrophobic coating simultaneously, and it is possible to achieve simplification of the manufacturing process.

It is preferable for the force by which the sheath for introducer and the dilator are attached to be smaller than the force which acts on the attachment region when the dilator is pulled out from the sheath for introducer. In this case, by employing a configuration in which the attachment region is breakable in conjunction with the operation of pulling out the dilator, it is possible to handle the introducer assembly while maintaining similar feeling as heretofore and it never brings uncomfortable feeling to the user.

It is preferable to attach the sheath for introducer and the dilator by reactively curing the coating material depending on heat at the time of gas sterilization. In this case, it is possible to carry out the formation of the hydrophobic coating and the attachment between the sheath for introducer and the dilator through this hydrophobic coating in a packed state simultaneously at the time of gas sterilization, and it is possible to achieve simplification of the manufacturing process.

Still other objects, features and characteristics of the present invention will become clear by referring to preferable exemplified embodiments illustrated in the explanation below and attached drawings.

### Brief Description of the Drawings

FIG. 1 is a plan view showing a state in which an introducer assembly relating to an exemplified embodiment of the present invention is packaged inside a packaging film;
FIG. 2 is a plan view showing in an exploded manner a sheath for introducer and a dilator of an introducer assembly;
FIG. 3 is a cross-sectional view showing a sheath for introducer of a first exemplified embodiment;
FIG. 4A is a cross-sectional view showing in an enlarged manner a portion surrounded by a dashed-dotted line and applied with a reference numeral 4A in FIG. 3;
FIGS. 4B is a cross-sectional view equivalent to FIG. 4A showing a state after a sheath for introducer is inserted inside a blood vessel;
FIG. 5 is a cross-sectional view showing a sheath for introducer of an improved & modified example of the first exemplified embodiment;
FIG. 6 is a cross-sectional view showing a sheath for introducer of a second exemplified embodiment;
FIG. 7 is a cross-sectional view showing a sheath for introducer and a dilator in an introducer assembly of a third exemplified embodiment; and
FIG. 8 is a cross-sectional view showing a sheath for introducer of a fourth exemplified embodiment.

### Description of the Preferred Emboliments

Hereinafter, with reference to the attached drawings, an exemplified embodiment of the present invention will be explained. Note that, in explanations of the drawings, the same elements will be given the same reference numerals, and repetitive explanation will be omitted. Also, there is a case in which the size ratio of a drawing is exaggerated for convenience of explanation and it is different from the actual ratio.

### (First Exemplified Embodiment)

An introducer assembly 10 is a device for securing an access route to the inside of a body-cavity. Note that in the following explanation, the hand-side operation unit side of the device is referred to as "proximal side", and the side to be inserted into the inside of the body-cavity is referred to as "distal side".

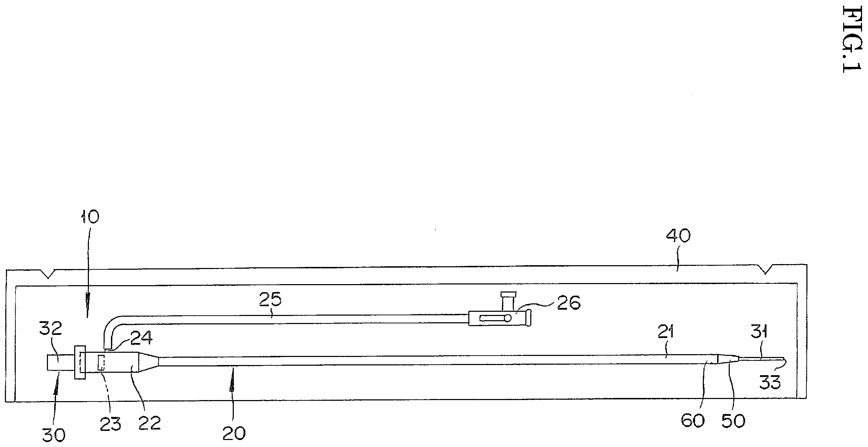

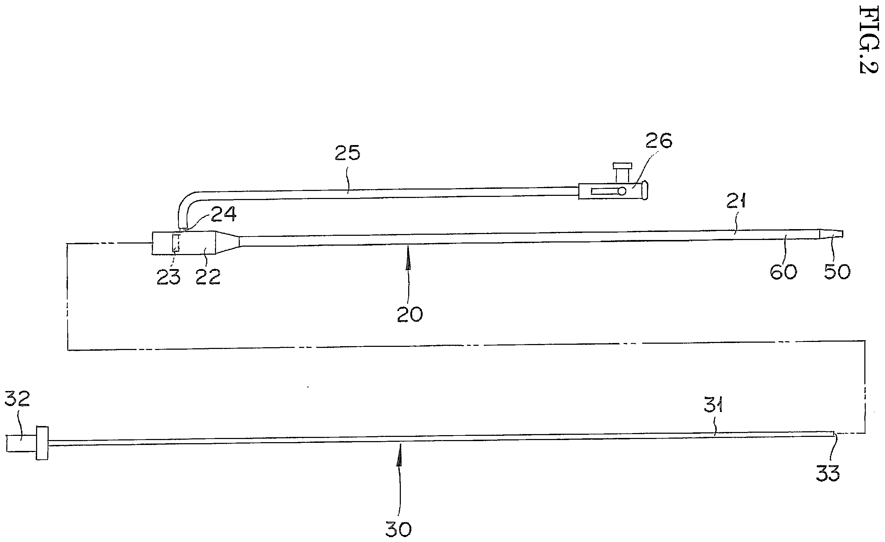

FIG. 1 is a plan view showing a state in which the introducer assembly 10 relating to an exemplified embodiment of the present invention is packaged inside a packaging film 40, and FIG. 2 is a plan view showing in an exploded manner a sheath 20 for introducer and a dilator 30 of the introducer assembly 10.

When roughly explained with reference to FIG. 1 and FIG. 2, the introducer assembly 10 includes the sheath for introducer 20 and the dilator 30. In this exemplified embodiment, the sheath for introducer 20 and the dilator 30 are integrated beforehand and packaged inside the packaging film 40. The sheath for introducer 20 is provided with a sheath tube 21, a sheath hub 22 attached to the proximal side of the sheath tube 21, and a homostasis valve 23 attached to the proximal side of the sheath hub 22. The dilator 30 is provided with a dilator tube 31 and a dilator hub 32 attached to the proximal side of the dilator tube 31. Hereinafter, the introducer assembly 10 will be described in detail.

The sheath for introducer 20 is indwelled inside the body-cavity and is a sheath for being introduced into the inside of the body-cavity, with an elongated body such as a catheter, a guide wire, an embolus object or the like being inserted therethrough in the inside thereof.

The sheath tube 21 is introduced percutaneously into the inside of the body-cavity.

As the constituent material of the sheath tube 21, it is possible to use, for example, a polymer material such as a polyolefin (e.g. polyethylene, polypropylene, polybutene, ethylene-propylene copolymer, ethylene-vinyl acetate copolymer, ionomer, a mixture of two kinds or more of these, or the like), polyolefin elastomer, a cross-linked body of polyolefin, polyvinyl chloride, polyamide, polyamide elastomer, polyester, polyester elastomer, polyurethane, polyurethane elastomer, fluororesin, polycarbonate, polystyrene, polyacetal, polyimide, polyetherimide or the like, a mixture of these, or the like. It is possible to use ethylenetetrafluoroethylene copolymer (ETFE) favorably.

A side port 24 which communicates with the inside of the sheath tube 21 is formed at the sheath hub 22. One end of a tube 25 having flexibility, which is made, for example, of polyvinyl chloride, is connected to the side port 24 in a liquid-tight manner. On the other end of the tube 25, for example, a T-shape stopcock 26 is mounted. A liquid such as physiological saline is injected into the sheath for introducer 20 from a port of this T-shape stopcock 26 through the tube 25.

For the constituent material of the sheath hub 22, there is no limitation in particular, but a hard material such as a hard resin is preferably used. As specific examples of the hard resin, it is possible to cite, for example, polyolefins such as polyethylene, polypropylene and the like, polyamides, polycarbonates, polystyrenes, and the like.

The homostasis valve 23 is constituted by an elastic member forming approximately an elliptical membrane shape (disc shape) and is fixed in a liquid-tight manner with respect to the sheath hub 22.

For the constituent material of the homostasis valve 23, there is no limitation in particular, but it is possible to cite, for example, silicone rubber, latex rubber, butyl rubber, isoprene rubber or the like, which is an elastic member.

The dilator 30 is used, when inserting the sheath for introducer 20 into the blood vessel, in order to prevent breakage of the sheath tube 21, in order to expand the diameter of a trephination in the skin, and so on.

The dilator tube 31 is inserted inside the sheath tube 21. As shown in FIG. 1, there is created a state in which the distal end 33 of the dilator tube 31 projects from the distal end of the sheath tube 21.

As the constituent material of the dilator tube 31, it is possible to use, for example, a polymer material such as polyolefin (e.g. polyethylene, polypropylene, polybutene, ethylene-propylene copolymer, ethylene-vinyl acetate copolymer, ionomer, a mixture of two kinds or more of these, or the like), polyolefin elastomer, a cross-linked body of polyolefin, polyvinyl chloride, polyamide, polyamide elastomer, polyester, polyester elastomer, polyurethane, polyurethane elastomer, fluororesin, polycarbonate, polystyrene, polyacetal, polyimide, polyetherimide or the like, a mixture of these, or the like.

The dilator hub 32 is held detachably with respect to the sheath hub 22.

For the constituent material of the dilator hub 32, there is no limitation in particular, but a hard material such as a hard resin is favorably used. As specific examples of the hard resin, it is possible to cite, for example, polyolefins such as polyethylene, polypropylene and the like, polyamides, polycarbonates, polystyrenes, and the like.

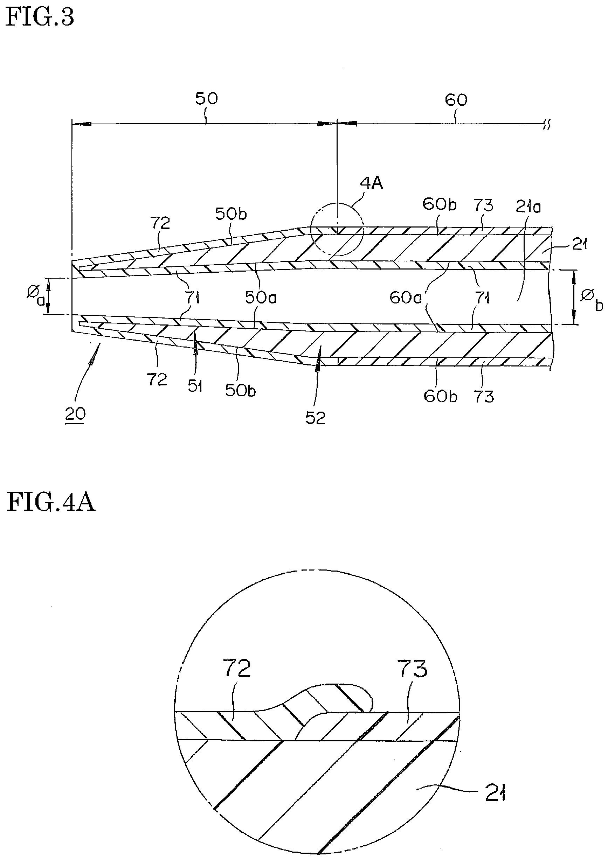

FIG. 3 is a cross-sectional view showing a sheath for introducer 20 of a first exemplified embodiment, and FIG. 4A is a cross-sectional view showing in an enlarged manner a portion surrounded by a dashed-dotted line and given a reference numeral 4A in FIG. 3.

When roughly explained with reference to FIG. 3, the sheath for introducer 20 of the first exemplified embodiment is formed by the sheath tube 21 (corresponding to the tubular member) provided with a hollow portion 21a through which an elongated body such as a catheter is freely insertable, and the sheath is provided with a sheath distal portion 50 (corresponding to distal portion) and a sheath main body portion 60 (corresponding to main body portion). The sheath distal portion 50 includes a taper portion 51 which tapers and a straight portion 52 which extends approximately in parallel with the axis line.

As for the sheath for introducer 20, the inner diameter Φa of the sheath distal portion 50 is formed to become gradually smaller toward distal side thereof. The sheath for introducer 20 is further provided, on the inner surface 50a of the sheath distal portion 50, with a hydrophobic coating 71 having a friction coefficient lower than the friction coefficient of the sheath tube 21. In the illustrated example, there is provided a hydrophobic coating 71 over the whole lengths of the inner surface 50a of the sheath distal portion 50 and the inner surface 60a of the sheath main body portion 60.

On the outer surface 50b of the sheath distal portion 50, there is further provided a hydrophobic coating 72. The hydrophobic coating 72 will be described later.

The sheath for introducer 20 is provided with a hydrophilic lubricating coating 73 on the outer surface 60b of the sheath main body portion 60. The sheath for introducer 20 is further provided, on the outer surface 50b of the sheath distal portion 50, with the hydrophobic coating 72 having a friction coefficient lower than the friction coefficient of the hydrophilic lubricating coating 73 during the dry period thereof.

Here, the term "dry" broadly means a state in which the hydrophilic lubricating coating 73 is not wet and can not exert sufficient lubricity. Therefore, it is not necessary to specify the degree of "dry" according to the temperature and the humidity.

Regarding the size of the sheath for introducer 20, in a case in which the size of the catheter is 6Fr, the length of the sheath distal portion 50 is 3mm to 4mm, the inner diameter Φa of the most distal end is 1. 98mm to 2.13mm and the inner diameter Φb of the sheath main body portion 60 is 2.22mm. It is needless to say that the size of the sheath for introducer 20 is not limited to the abovementioned dimensions.

The hydrophobic coatings 71, 72 are formed by being coated with a hydrophobic material. For the hydrophobic material, it is possible to cite, for example, reactive-curing silicone, polytetrafluoroethylene (PTFE), fluorinatedethylenepropylene (FEP) or the like.

The hydrophilic lubricating coating 73 is formed by coating a hydrophilic material. As the hydrophilic material, it is possible to cite, for example, an acrylamide-based polymer material (e.g., polyacrylamide, a block copolymer of polyglycidylmethacrylate-dimethylacrylamide (PGMA-DMAA)), a cellulose-based polymer material, a polyethylene oxide-based polymer material, a maleic anhydride-based polymer material (e.g., a maleic anhydride copolymer such as methylvinylether-maleic anhydride copolymer), water-soluble nylon, polyvinyl alcohol, polyvinylpyrrolidone or the like.

It is necessary for the hydrophobic coating 72 on the outer surface 50b at the sheath distal portion 50 to be provided at least so as to cover the taper portion 51 of the sheath distal portion 50. The taper portion 51 of the sheath distal portion 50 is a region to which the greatest resistance is applied at the time of insertion. Consequently, when the taper portion 51 which is not provided with the hydrophobic coating 72 is exposed to the outer surface 50b, there occurs a phenomenon that reduction in insertion resistance is hampered and this is the reason for the necessity thereof. It is desirable for the region provided with the hydrophobic coating 72 within the straight portion 52 of the sheath distal portion 50 to have a range of less than 1/2 of the effective length of the sheath for introducer 20. This is derived in consideration of a balance between the resistance reduction effect at the time when the sheath for introducer 20 is inserted and the resistance reduction effect at the time when it is pulled out.

In a case in which the hydrophobic coatings 71, 72 of the inner surface 50a and the outer surface 50b of the sheath distal portion 50 are formed by reactive-curing silicone, the hydrophobic coatings 71, 72 themselves cure and the sheath distal portion 50 cures, thereby making it possible to suppress a turnup of the sheath distal portion 50.

The sheath for introducer 20 is manufactured as follows.

First, the hydrophilic lubricating coating 73 is formed only on the outer surface 60b of the sheath main body portion 60 of the sheath for introducer 20. A cored bar is inserted through the sheath tube 21 and there is coating with a hydrophilic material, and the hydrophilic lubricating coating 73 is formed. As the hydrophilic material, for example, polyglycidylmethacrylate-dimethylacrylamide (PGMA-DMAA) or the like is used. As the material of the sheath tube 21, for example, ethylenetetrafluoroethylene copolymer (ETFE) or the like is used.

Subsequently, a process of shape-application on the sheath distal portion 50 is carried out. For this process, there is used a die assembly in which there is formed a recess having an inner-surface shape which conforms to the taper shape of the sheath distal portion 50. The die assembly is heated by a high frequency power supply. The distal end of the sheath tube 21 is pressed into the recess of the die assembly. Then, the inner-surface shape of the recess is transferred to the distal end of the sheath tube 21 and at the sheath distal portion 50, there is formed the taper portion 51 at which the outer surface 50b tapers.

Subsequently, the hydrophobic coating 71 is formed over the whole lengths of the inner surface 50a of the sheath distal portion 50 and the inner surface 60a of the sheath main body portion 60. The inner surfaces 50a, 60a of the sheath for introducer 20 are coated with a hydrophobic material and the hydrophobic coating 71 is formed. Further, the hydrophobic coating 72 is also formed on the outer surface 50b of the sheath distal portion 50. The sheath distal portion 50 is dipped into a hydrophobic material and the hydrophobic coating 72 is formed. As the hydrophobic material, for example, reactive-curing silicone or the like is used. As this hydrophobic material, a material is used which has a friction coefficient lower than the friction coefficient of the hydrophilic lubricating coating 73 during the dry period thereof. In the abovementioned example, reactive-curing silicone having a friction coefficient lower than the friction coefficient of ETFE during the dry period thereof is used as the hydrophobic material.

By way of the processes mentioned above, the sheath for introducer 20 shown in FIG. 3 is formed.

For the sheath for introducer 20 of the first exemplified embodiment, the inner diameter Φa of the most distal end thereof is formed to be smaller than the outer diameter of the elongated body such as a catheter, but on the inner surface 50a of the sheath distal portion 50, there is provided the hydrophobic coating 71 (coating composed of reactive-curing silicone) having a friction coefficient lower than the friction coefficient of the raw material (e.g. , ETFE) of the sheath tube 21. Consequently, the sliding resistance between the inner surface 50a of the sheath distal portion 50 and the elongated body such as a catheter is lowered and it is possible to achieve improvement in the slidability of the catheter or the like.

Here, in a case in which a hydrophilic lubricating coating is formed on the inner surface 50a of the sheath distal portion 50, a process for activating the raw material of the sheath tube 21 becomes necessary and the hydrophilic lubricating coating must be formed before carrying out the shape-application process on the sheath distal portion 50. Consequently, the heat at the time of carrying out the shape-application process on the sheath distal portion 50 by using the die assembly acts excessively and there occurs a phenomenon that the hydrophilic coating peels, decomposes, degrades or the like. Consequently, the sliding resistance between the inner surface 50a of the sheath distal portion 50 and the elongated body such as a catheter does not lower substantially and it is not possible to achieve improvement in the slidability of the catheter or the like.

On the other hand, in case of forming the hydrophobic coating 71 on the inner surface 50a of the sheath distal portion 50, it can be formed by coating with a hydrophobic material after carrying out the shape-application process on the sheath distal portion 50, and thus peeling, decomposition, degradation and the like of the hydrophobic coating 71 do not occur. Therefore, as mentioned above, the sliding resistance between the inner surface 50a of the sheath distal portion 50 and the elongated body such as a catheter is lowered and it is possible to achieve improvement in the slidability of the catheter or the like.

The sheath for introducer 20 is further provided with the hydrophobic coating 72 having a friction coefficient lower than the friction coefficient of the hydrophilic lubricating coating 73 during the dry period thereof, on the outer surface 50b of the sheath distal portion 50 to which the greatest resistance is applied at the time of insertion. The hydrophilic coating has a poor sliding property under a dry condition, but by providing the hydrophobic coating 72 on the outer surface 50b of the sheath distal portion 50, it is possible to lower the insertion resistance of the sheath for introducer 20 when inserting the sheath from the skin into the blood vessel. When pulling out the sheath for introducer 20 after its insertion into the blood vessel, the hydrophilic lubricating coating 73 becomes wet by the blood and exerts lubricity, so that it is possible to pull it out smoothly.

In this manner, according to the sheath for introducer 20 of the first exemplified embodiment, even in a case in which the inner diameter Φa of the sheath distal portion 50 is formed to become gradually smaller toward the distal side, it is possible to achieve improvement in the slidability of the elongated body such as a catheter. Furthermore, according to the sheath for introducer 20 of this exemplified embodiment, it is possible to achieve reduction in the insertion resistance even under a dry condition at the beginning of the use thereof.

FIG. 4A shows a structure in which the hydrophobic coating 72 on the outer surface 50b of the sheath distal portion 50 and the hydrophilic lubricating coating 73 on the outer surface 60b of the sheath main body portion 60 overlap each other.

As shown in FIG. 4A, at the boundary portion between the sheath distal portion 50 and the sheath main body portion 60, it is desirable to employ a structure in which the hydrophobic coating 72 lies over the hydrophilic lubricating coating 73. This is because at the time of insertion of the sheath for introducer 20 from the skin into the blood vessel, the sheath does not get stuck on the end surface of the hydrophilic lubricating coating 73 at the boundary portion. By employing such a structure, it is possible to insert the sheath for introducer 20 smoothly and it is even possible to suppress peeling of the hydrophilic lubricating coating 73.

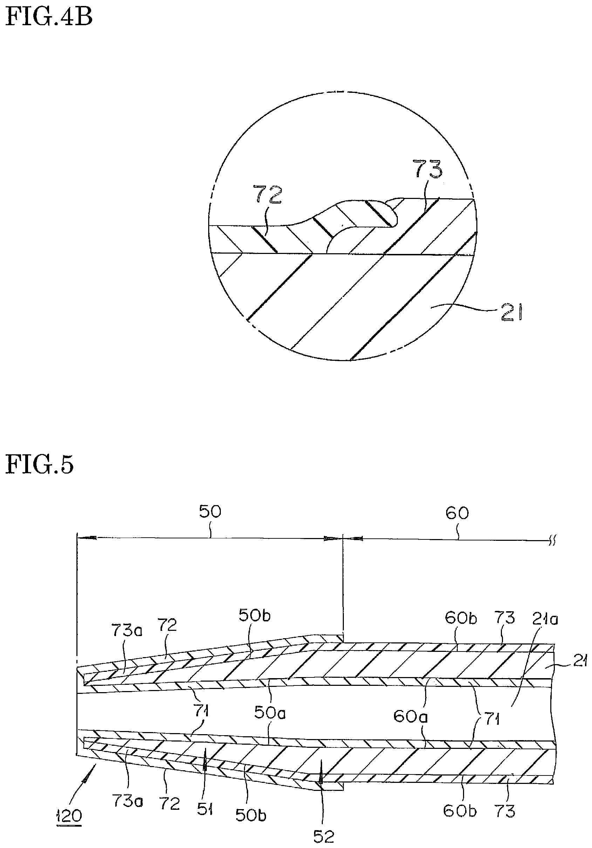

FIG. 4B shows the state of the hydrophilic lubricating coating 73 and the hydrophobic coating 72 after insertion of the sheath for introducer 20 into the blood vessel.

As shown in FIG. 4B, the hydrophilic lubricating coating 73 absorbs the blood and swells, and the diameter thereof becomes approximately equal to or larger than the outer diameter of the hydrophobic coating 72. According to this fact, when the sheath for introducer 20 is pulled out from the blood vessel, the overlapping portion, formed by a configuration in which the proximal portion of the hydrophobic coating 72 overlaps the distal portion of the hydrophilic lubricating coating 73, does not become a level difference. Therefore, the resistance of the surface of the sheath for introducer 20 with respect to the blood vessel and skin does not become excessive and it is possible to pull out the sheath for introducer 20 smoothly due to both the coatings 72 and 73.

According to the experiment, by providing the hydrophobic coating 71 formed from reactive-curing silicone, the sliding resistance of the inner surface 50a at the sheath distal portion 50 lowered by as much as approximately 80% compared with a case in which the hydrophobic coating 71 was not applied. Further, by providing the hydrophobic coating 72 formed from reactive-curing silicone, the penetration resistance of the outer surface 50b at the sheath distal portion 50 lowered by as much as approximately 30% compared with a case in which the hydrophilic lubricating coating 73 was applied. In this manner, it was confirmed by the experiment that high effectiveness can be obtained both in relation to reduction in the sliding resistance of the inner surface 50a at the sheath distal portion 50 and in relation to reduction in the penetration resistance of the outer surface 50b at the sheath distal portion 50.

### (Improved & Modified Example of First Exemplified Embodiment)

FIG. 5 is a cross-sectional view showing a sheath for introducer of an improved & modified example of the first exemplified embodiment.

The improved & modified example of the first exemplified embodiment is an example in which the manufacturing procedure of a sheath for introducer 120 is improved & modified with respect to the first exemplified embodiment mentioned above.

In the manufacturing procedure relating to the improved & modified example of the first exemplified embodiment, first, the hydrophilic lubricating coating 73 is formed not only on the outer surface 60b of the sheath main body portion 60 of the sheath for introducer 120 but also on the outer surface 50b of the sheath distal portion 50. A cored bar is inserted through the sheath tube 21 and with dipping into a hydrophilic material, the hydrophilic lubricating coating 73 is formed on the whole of the outer surfaces 50b, 60b of the sheath for introducer 120.

Subsequently, a shape-application process on the sheath distal portion 50 is carried out. Depending on the heat which acts when the shape-application process on the sheath distal portion 50 is carried out using a die assembly, the hydrophilic lubricating coating 73 on the outer surface 50b of the sheath distal portion 50 disappears, the lubricity thereof is deactivated although the coating itself remains and so on. The improved & modified example of the first exemplified embodiment shows the latter state in which the lubricity is deactivated although the coating itself remains. As shown in FIG. 5, the coating 73a whose lubricity has been deactivated remains on the outer circumference surface 50b of the sheath distal portion 50.

Subsequently, the hydrophobic coating 71 is formed over the whole lengths of the inner surface 50a of the sheath distal portion 50 and the inner surface 60a of the sheath main body portion 60. The inner surfaces 50a, 60a of the sheath for introducer 20 are coated with a hydrophobic material and the hydrophobic coating 71 is formed. Further, the hydrophobic coating 72 is formed also on the coating 73a, whose lubricity has been deactivated, at the sheath distal portion 50. By dipping the sheath distal portion 50 into a hydrophobic material, the hydrophobic coating 72 is formed.

By way of the processes mentioned above, the sheath for introducer 120 shown in FIG. 5 is formed. In the improved & modified example of the first exemplified embodiment, the hydrophobic coating 72 is formed on the coating 73a whose lubricity has been deactivated, so that functional groups remaining in the coating 73a are coupled with the hydrophobic coating. Consequently, it is possible to improve fixation property compared with a case in which a sheath made of ethylene-tetra-fluoro-ethylene copolymer (ETFE) is directly covered with a hydrophobic coating.

### (Second Exemplified Embodiment)

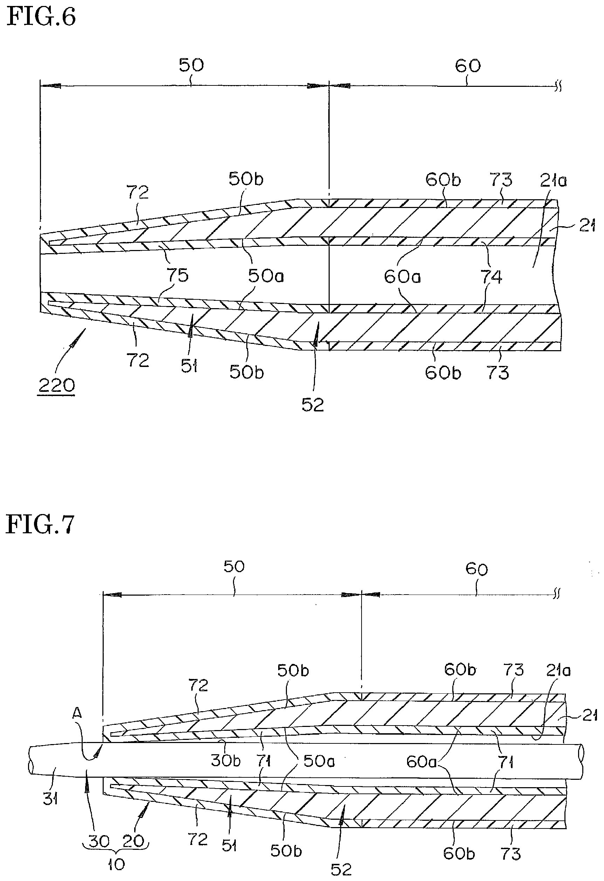

FIG. 6 is a cross-sectional view showing a sheath for introducer 220 of a second exemplified embodiment.

With reference to FIG. 6, the sheath for introducer 220 of the second exemplified embodiment is provided with a hydrophilic lubricating coating 74 on the inner surface 60a of the sheath main body portion 60 and in terms of this aspect, it is different from the sheath for introducer 20 of the first exemplified embodiment which is provided with the hydrophobic coating 71 on the inner surface 60a of the sheath main body portion 60.

Regarding the sheath for introducer 220 of the second exemplified embodiment, similarly to the first exemplified embodiment, the inner diameter Φa of the sheath distal portion 50 thereof is formed to become gradually smaller toward the distal side. On the inner surface 50a of the sheath distal portion 50, there is provided a hydrophobic coating 75 having a friction coefficient lower than the friction coefficient of the sheath tube 21. Also, for the sheath for introducer 220, similarly to the first exemplified embodiment, the hydrophilic lubricating coating 73 is provided on the outer surface 60b of the sheath main body portion 60, and the hydrophobic coating 72 having a friction coefficient lower than the friction coefficient of the hydrophilic lubricating coating 73 during the dry period thereof is provided on the outer surface 50b of the sheath distal portion 50.

The size, the hydrophobic material and the hydrophilic material of the sheath for introducer 220 are similar to those of the first exemplified embodiment.

The sheath for introducer 220 is manufactured as follows.

First, the inner surfaces 50a, 60a and the outer surfaces 50b, 60b of the sheath for introducer 220 are subjected to plasma treatment and a process for activating the raw-material surface layer of the sheath tube 21 is carried out.

Subsequently, the hydrophilic lubricating coatings 73, 74 are formed on the inner surfaces 50a, 60a and the outer surfaces 50b, 60b of the sheath for introducer 220. By dipping the inner surfaces 50a, 60a and the outer surfaces 50b, 60b of the sheath tube 21 into a hydrophilic material, the hydrophilic lubricating coating 73 is formed. As the hydrophilic material, for example, polyglycidylmethacrylate-dimethylacrylamide (PGMA-DMAA) or the like is used. As the material of the sheath tube 21, for example, ethylenetetrafluoroethylene copolymer (ETFE) or the like is used.

Subsequently, a shape-application process on the sheath distal portion 50 is carried out. In this process, a die assembly is used in which there is formed a recess having an inner-surface shape which conforms to the taper shape of the sheath distal portion 50. The die assembly is heated by a high frequency power supply. The distal end of the sheath tube 21 is pressed into the recess of the die assembly. Then, the inner-surface shape of the recess is transferred to the distal end of the sheath tube 21, and at the sheath distal portion 50 there is formed the taper portion 51 at which the outer surface 50b tapers. In the former process, at the time of dipping into the hydrophilic material, the hydrophilic lubricating coating is formed on the inner surface 50a and the outer surface 50b of the sheath distal portion 50. However, depending on the heat which acts at the time of carrying out the shape-application process on the sheath distal portion 50 by using the die assembly, the hydrophilic lubricating coating on the inner surface 50a and the outer surface 50b of the sheath distal portion 50 disappears, the lubricity thereof is deactivated although the coating itself remains and so on. Note that the second exemplified embodiment shows the former state in which the hydrophilic lubricating coating has disappeared.

Subsequently, the hydrophobic coatings 75, 72 are formed on the inner surface 50a and the outer surface 50b of the sheath distal portion 50. The hydrophobic coatings 75, 72 are formed at the portions at which the hydrophilic lubricating coating has disappeared. By dipping the sheath distal portion 50 into a hydrophobic material, the hydrophobic coatings 75, 72 are formed. As the hydrophobic material, for example, reactive-curing silicone or the like is used. For this hydrophobic material, a material is used which has a friction coefficient lower than the friction coefficient of the hydrophilic lubricating coating 73 during the dry period thereof. In the abovementioned example, reactive-curing silicone having a friction coefficient lower than the friction coefficient of ETFE during the dry period thereof is used as the hydrophobic material.

By way of the processes mentioned above, the sheath for introducer 220 shown in FIG. 6 is formed.

For the sheath for introducer 220 of the second exemplified embodiment, similarly to the first exemplified embodiment, the inner diameter Φa of the most distal end thereof is formed to be smaller than the outer diameter of the elongated body such as a catheter, but on the inner surface 50a of the sheath distal portion 50, there is provided the hydrophobic coating 75 (coating composed of reactive-curing silicone) having a friction coefficient lower than the friction coefficient of the raw material (e.g., ETFE) of the sheath tube 21. Consequently, the sliding resistance between the inner surface 50a of the sheath distal portion 50 and the elongated body such as a catheter is lowered, which makes it possible to achieve improvement in the slidability of the catheter or the like.

Also, in case of forming the hydrophobic coatings 75, 72 on the inner surface 50a and the outer surface 50b of the sheath distal portion 50, it is possible to form them by dipping into a hydrophobic material after carrying out the shape-application process on the sheath distal portion 50, and peeling, decomposition, degradation and the like of the hydrophobic coatings 75, 72 do not occur. Therefore, as mentioned above, the sliding resistance between the inner surface 50a of the sheath distal portion 50 and the elongated body such as a catheter is lowered, in which it is possible to achieve improvement in the slidability of the catheter or the like.

The sheath for introducer 220 is further provided with the hydrophobic coating 72 having a friction coefficient lower than the friction coefficient of the hydrophilic lubricating coating 73 during the dry period thereof, on the outer surface 50b of the sheath distal portion 50. The hydrophilic coating has a poor sliding property under a dry condition, but by providing the hydrophobic coating 72 on the outer surface 50b of the sheath distal portion 50, it is possible to lower the insertion resistance of the sheath for introducer 220 when inserting it from the skin into the blood vessel. When the sheath for introducer 220 is pulled out after its insertion it into the blood vessel, the hydrophilic coating becomes wet by the blood and exerts lubricity, so that it is possible to pull it out smoothly.

In this manner, according to the sheath for introducer 220 of the second exemplified embodiment, even in a case in which the inner diameter Φa of the sheath distal portion 50 is formed to become gradually smaller toward the distal side, it is possible to achieve improvement in the slidability of the elongated body such as a catheter and furthermore, it is possible to achieve reduction in insertion resistance even under a dry condition at the beginning of the use thereof.

Similarly to the first exemplified embodiment, it is desirable for the boundary portion between the sheath distal portion 50 and the sheath main body portion 60 to employ a structure in which the hydrophobic coating 72 lies over the hydrophilic lubricating coating 73. This is because it is possible to insert the sheath for introducer 220 smoothly and it is also possible to suppress peeling of the hydrophilic lubricating coating 73.

### (Third Exemplified Embodiment)

FIG. 7 is a cross-sectional view showing the sheath for introducer 20 and the dilator 30 in the introducer assembly 10 of a third exemplified embodiment.

The introducer assembly 10 is constituted such that the dilator 30 as an elongated body is inserted through the sheath for introducer 20 of the first exemplified embodiment and the distal end 33 of the dilator 30 projects from the sheath distal portion 50. In this introducer assembly 10, at least a portion of the inner surface 50a of the sheath distal portion 50 and a portion of the outer surface 30b of the dilator 30 are attached to each other through the hydrophobic coating 71. In the drawing, the sheath distal portion 50 and the dilator 30 are attached to each other at the portion indicated by a reference numeral A.

By attaching the sheath distal portion 50 and the dilator 30 to each other, a turnup of the sheath distal portion 50 is suppressed when the sheath for introducer 20 is inserted from the skin into the blood vessel, whereby it is possible to insert the sheath for introducer 20 smoothly.

There is no limitation for the region at which the inner surface 50a of the sheath distal portion 50 and the outer surface 30b of the dilator 30 are attached to each other, but from a view point of suppressing a turnup of the sheath distal portion 50, it is preferable to perform the attachment at the position which nears the distal end side as much as possible.

It is possible for the hydrophobic coating 71 to be formed by a reactive-curing coating material, for example reactive-curing silicone. In this case, it is desirable for the hydrophobic coating 71 to be formed by reactively curing the coating material in a state in which the dilator 30 is inserted through the sheath for introducer 20. This is because, according to such an introducer assembly 10, it is possible to simultaneously carry out formation of the hydrophobic coating 71 and attachment between the inner surface 50a of the sheath distal portion 50 and the outer surface 30b of the dilator 30 through this hydrophobic coating 71 and it is possible to achieve simplification of the manufacturing process.

Further, by forming the hydrophobic coatings 71, 72 on the inner surface 50a and the outer surface 50b of the sheath distal portion 50 from the curing reactive silicone, the hydrophobic coatings 71, 72 themselves cure, and the sheath distal portion 50 cures, which makes it possible to suppress a turnup of the sheath distal portion 50 even further.

In the introducer assembly 10, it is desirable for the force, by which the sheath for introducer 20 and the dilator 30 are attached to each other, to be smaller than the force which acts on the attachment region when the dilator 30 is pulled out from the sheath for introducer 20.

This is because, by configuring the attachment region to be a breakable, it is possible to deal with the introducer assembly 10 while maintaining similar feelings felt until now, which does not give uncomfortable feelings to the operator.

The introducer assembly 10 makes it possible to attach the sheath for introducer 20 and the dilator 30 to each other by reactively curing a coating material depending on the heat at the time of gas sterilization.

This is because, according to such an introducer assembly 10, it is possible to simultaneously carry out formation of the hydrophobic coating 71 and attachment of the sheath for introducer 20 and the dilator 30 to each other through this hydrophobic coating 71 in a packed state at the time of EOG sterilization, whereby it is possible to achieve simplification of the manufacturing process.

### (Fourth Exemplified Embodiment)

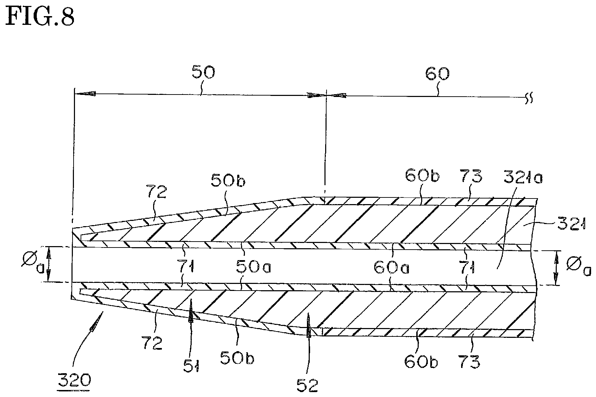

FIG. 8 is a cross-sectional view showing a sheath for introducer 320 of a fourth exemplified embodiment.

With respect to the sheath for introducers 20, 120, 220 mentioned above, the inner diameter Φa of the sheath distal portion 50 is formed to become gradually smaller toward the distal side and on the inner surface 50a of the sheath distal portion 50, and there is formed the hydrophobic coating 71 having a friction coefficient lower than the friction coefficient of the sheath tube 21. It is possible to lower the sliding resistance of the inner surface 50a by providing the hydrophobic coating 71, so that it becomes possible to achieve reduction in sliding resistance even if the contact area with respect to the catheter or the like increases.

Then, as for the sheath for introducer 320 of the fourth exemplified embodiment, there is employed a configuration in which the inner diameter of the sheath distal portion 50 is formed with uniform size without diameter reduction. In terms of this aspect, the fourth exemplified embodiment is different from the first to third exemplified embodiments.

In more detail, as shown in FIG. 8, the sheath for introducer 320 according to the fourth exemplified embodiment is formed by a sheath tube 321 (corresponding to tubular member) provided with a hollow portion 321a through which the elongated body such as a catheter is freely insertable. The sheath for introducer 320 is provided with the sheath distal portion 50 (corresponding to distal portion) and the sheath main body 60 (corresponding to main body portion). The sheath distal portion 50 includes a taper portion 51 which tapers and a straight portion 52 which extends approximately in parallel with the axial line.

As for the sheath for introducer 320, the inner diameter of the sheath distal portion 50 is not reduced and is formed uniformly as the inner diameter Φa. Also the inner diameter of the sheath main body 60 is formed uniformly as the inner diameter Φa. The sheath for introducer 320 is further provided, on the inner surface 50a of the sheath distal portion 50, with the hydrophobic coating 71 having a friction coefficient lower than the friction coefficient of the sheath tube 321. In this fourth exemplified embodiment, there is provided the hydrophobic coating 71 over the whole lengths of the inner surface 50a of the sheath distal portion 50 and the inner surface 60a of the sheath main body 60.

With respect to an aspect in which there is provided, on the outer surface 60b sheath main body 60, the hydrophilic lubricating coating 73; an aspect in which there is provided, on the outer surface 50b of the sheath distal portion 50, the hydrophobic coating 72 having a friction coefficient lower than the friction coefficient of the hydrophilic lubricating coating 73 during the dry period thereof; an aspect of the constituent materials of the sheath tube 321; an aspect of the constituent materials of the hydrophobic coatings 71, 72; and an aspect of the constituent materials of the lubricating coating 73, they are similar to those explained in the first exemplified embodiment. As for the manufacturing procedure of the sheath for introducer 320, it is similar to that explained in the first exemplified embodiment except an aspect in which a straight-shape cored bar is inserted through the sheath tube 321 such that the inner diameter of the sheath tube 321 is not reduced when carrying out the shape-application process on the sheath distal portion 50.

According to the sheath for introducer 320 of the fourth exemplified embodiment, it is possible to achieve improvement in the slidability of the elongated body such as a catheter even in case of forming the inner diameter of the sheath tube 321 uniformly. Furthermore, according to the sheath for introducer 320, it is possible to achieve reduction in insertion resistance even under a dry condition at the beginning of the use thereof.

As mentioned above, the sheaths for introducer 20, 120, 220, 320 and the introducer assembly 10 of the present invention were explained based on the exemplified embodiments shown in the drawings, but the present invention is not to be limited by these aspects.

For example, in a case in which reduction in the sliding resistance of the inner surface 50a in the sheath distal portion 50 is to be mainly achieved, it is enough if the inner diameter Φa of the sheath distal portion 50 is formed to become gradually smaller toward the distal side and only the hydrophobic coating 71 having a friction coefficient lower than the friction coefficient of the sheath tube 21 is formed only on the inner surface 50a of the sheath distal portion 50.

Also, in a case in which reduction in the penetration resistance of the outer surface 50b in the sheath distal portion 50 is to be mainly achieved, it is enough if the hydrophilic lubricating coating 73 is provided on the outer surface 60b of the sheath main body portion 60 and the hydrophobic coating 72 having a friction coefficient lower than the friction coefficient of the hydrophilic lubricating coating 73 during the dry period thereof is formed on the outer surface 50b of the sheath distal portion 50. In this case, the hydrophobic coating of the inner surface 50a in the sheath distal portion 50 is not indispensable.

### Explanation of Reference Symbols

- 10:: introducer assembly,
- 20, 120, 220:: sheath for introducer,
- 21:: sheath tube (tubular member),
- 21a:: hollow portion,
- 30:: dilator,
- 30b:: outer surface of dilator,
- 33:: distal end,
- 50:: sheath distal portion (distal portion),
- 50a:: inner surface of sheath distal portion,
- 50b:: outer surface of sheath distal portion,
- 60:: sheath main body portion (main body portion),
- 60a:: inner surface of sheath main body portion,
- 60b:: outer surface of sheath main body portion,
- 71, 72, 75:: hydrophobic coating,
- 73, 74:: lubricating coating of hydrophilic,
- 73a:: coating in which lubricity is deactivated,
- 321:: sheath tube (tubular member)
- 321a:: hollow portion
- Φa:: inner diameter of sheath distal portion.

## Claims

1. A sheath for introducer (20, 120, 220, 320), which is formed from a tubular member (21, 321) provided with a hollow portion (21a, 321a) through which an elongated body is freely insertable, and which includes a distal portion (50) and a main body portion (60), wherein
there is a hydrophilic lubricating coating (73) on the outer surface (60b) of the main body portion (60), and
on the outer surface (50b) of the distal portion (50), there is a hydrophobic coating (72) having a friction coefficient lower than the friction coefficient of the hydrophilic lubricating coating (73) in the dry state thereof,
the distal portion (50) includes a taper portion (51); and
the hydrophobic coating (72) on the outer surface (50b) of the distal portion (50) is provided at least so as to cover the taper portion (51).

2. The sheath for introducer (20, 120, 220, 320) according to claim 1, wherein at the boundary portion between the distal portion (50) and the main body portion (60), the hydrophobic coating (72) lies over the hydrophilic lubricating coating (73).

3. The sheath for introducer (20, 120, 220, 320) according to claim 2, wherein when being swollen by absorbed blood, the hydrophilic lubricating coating (73) has a diameter approximately equal to or greater than the outer diameter of the hydrophobic coating (72).

4. The sheath for introducer (20, 120, 220, 320) according to any of claims 1 to 3, wherein the sheath for introducer (20, 120, 220) is further provided, on the inner surface (50a) of the sheath distal portion (50), with a hydrophobic coating (71) having a friction coefficient lower than the friction coefficient of the tubular member (21, 321).

## Patentansprüche

1. Hülle für eine Einführungsvorrichtung (20, 120, 220, 320), die aus einem röhrenförmigen Element (21, 321) gebildet ist, das mit einem hohlen Abschnitt (21a, 321a) versehen ist, durch welchen ein länglicher Körper frei einsetzbar ist, und die einen distalen Abschnitt (50) und einen Hauptkörperabschnitt (60) umfasst, wobei
auf der äußeren Oberfläche (60b) des Hauptkörperabschnitts (60) eine hydrophile Gleitbeschichtung (73) vorhanden ist, und
auf der äußeren Oberfläche (50b) des distalen Abschnitts (50) eine hydrophobe Beschichtung (72) mit einem Reibungskoeffizienten vorhanden ist, der niedriger als der Reibungskoeffizient der hydrophilen Gleitbeschichtung (73) in dem trockenen Zustand davon ist,
der distale Abschnitt (50) einen sich verjüngenden Abschnitt (51) umfasst; und
die hydrophobe Beschichtung (72) an der äußeren Oberfläche (50b) des distalen Abschnitts (50) so vorgesehen ist, dass er mindestens den sich verjüngenden Abschnitt (51) bedeckt.

2. Hülle für eine Einführungsvorrichtung (20, 120, 220, 320) nach Anspruch 1, wobei in dem Grenzabschnitt zwischen dem distalen Abschnitt (50) und dem Hauptkörperabschnitt (60) die hydrophobe Beschichtung (72) über der hydrophilen Gleitschicht (73) liegt.

3. Hülle für eine Einführungsvorrichtung (20, 120, 220, 320) nach Anspruch 2, wobei, wenn sie durch Absorption von Blut aufgequollen ist, die hydrophile Gleitbeschichtung (73) einen Durchmesser aufweist, der annähernd gleich zu oder größer als der Außendurchmesser der hydrophoben Beschichtung (72) ist.

4. Hülle für eine Einführungsvorrichtung (20, 120, 220, 320) nach einem der Ansprüche 1 bis 3, wobei die Hülle für die Einführungsvorrichtung (20, 120, 220) ferner auf der inneren Oberfläche (50a) des distalen Hüllenabschnitts (50) mit einer hydrophoben Beschichtung (71) versehen ist, die einen Reibungskoeffizienten aufweist, der niedriger als der Reibungskoeffizient des röhrenförmigen Elements (21, 321) ist.

## Revendications

1. Gaine pour introducteur (20, 120, 220, 320), qui est formée d'un élément tubulaire (21, 321) muni d'une partie creuse (21a, 321a) à travers laquelle un corps allongé peut être librement inséré, et qui comprend une partie distale (50) et une partie de corps principal (60), dans laquelle
un revêtement lubrifiant hydrophile (73) est prévu sur la surface extérieure (60b) de la partie de corps principal (60), et
sur la surface extérieure (50b) de la partie distale (50), se trouve un revêtement hydrophobe (72) ayant un coefficient de frottement inférieur au coefficient de frottement du revêtement lubrifiant hydrophile (73) dans son état sec,
la partie distale (50) comprend une partie conique (51) ; et
le revêtement hydrophobe (72) sur la surface extérieure (50b) de la partie distale (50) est prévu de manière à couvrir au moins la partie conique (51).

2. Gaine pour introducteur (20, 120, 220, 320) selon la revendication 1, dans laquelle au niveau de la partie limite entre la partie distale (50) et la partie de corps principal (60), le revêtement hydrophobe (72) est placé sur le revêtement lubrifiant hydrophile (73).

3. Gaine pour introducteur (20, 120, 220, 320) selon la revendication 2, dans laquelle, lorsqu'il est gonflé par le sang absorbé, le revêtement lubrifiant hydrophile (73) a un diamètre approximativement supérieur ou égal au diamètre extérieur du revêtement hydrophobe (72).

4. Gaine pour introducteur (20, 120, 220, 320) selon l'une quelconque des revendications 1 à 3, dans laquelle la gaine pour introducteur (20, 120, 220) est en outre munie, sur la surface intérieure (50a) de la partie distale de gaine (50), d'un revêtement hydrophobe (71) ayant un coefficient de fiction inférieur au coefficient de frottement de l'élément tubulaire (21, 321).
